# EUROPEAN PATENT SPECIFICATION

(11) **EP 2 143 378 B1**
(45) Date of publication and mention of the grant of the patent: **07.09.2011**
(21) Application number: 09165234.7
(22) Date of filing: 10.07.2009
(51) Int. Cl.: A61B 5/05, A61M 1/14, A61M 1/16, A61M 1/28

(54) **Dialysis cassette with conductivity sensor**
Dialysekassette mit Leitfähigkeitsensor
Cassette pour la dialyse avec un Capteur de conductivité

(30) Priority: 11.07.2008 IT BO20080436
(43) Date of publication of application: 13.01.2010
(73) Proprietor: Bellco S.r.l. con Unico Socio, Mirandola (IT)
(72) Inventor: Cianciavicchia, Domenico, 64040 Cavuccio (IT); Aldrovandi, Mauro, 41037 Mirandola (IT)
(74) Representative: Jorio, Paolo

(56) References cited:
- EP-A- 0 451 429
- EP-A- 0 820 776
- WO-A-2006/107728
- WO-A-2007/120812
- GB-A- 2 444 509
- US-A- 5 863 421

## Description

The present invention relates to a dialysis machine with a cassette of an improved type.

Dialysis is a method for blood purification capable of restoring hydrosaline equilibrium and eliminating the water in excess and the toxic substances that accumulate in the organism in the course of renal insufficiency, transferring them to a liquid with electrolytic content similar to that of normal plasma, which does not contain them; here and in what follows said liquid will be designated by the term "dialysing solution". The application of said method envisages that the blood, once drawn from the arm of the patient, will traverse the so-called arterial line and be introduced into the dialyser, at the outlet of which it traverses what is called the venous line and returns purified to the patient.

The dialysing solution used in dialysis does not contain those substances that it is desired to eliminate from the blood, such as urea, uric acid, creatinine, phosphorus, etc., whilst it contains a precise quantity of other substances that it is desired to rebalance, such as sodium, calcium, magnesium, potassium, etc.

Sodium is the electrolyte with the highest concentration in the extracellular compartment and hence in the blood. It may happen that, on account of the excessive loss of sodium from the extracellular space, there will be a corresponding intracellular hyperosmolarity, with recall of water within the cell and cellular hyperhydration (imbalance syndrome). This phenomenon, together with the process of ultrafiltration, i.e., of removal of water from the vascular compartment, reduces the capacity of the cardiocirculatory system to adapt to the reduced blood volume circulating, thus favouring onset of arterial hypotension. On the other hand, high sodium levels frequently lead to arterial hypertension, with marked increase in the sense of thirst, and in excessive interdialytic weight increases.

From what has been mentioned above, it is particularly important to be able to monitor the concentration of sodium in the blood during dialysis.

Whereas it is not possible to detect the concentration of sodium directly in the blood on account of the complex composition of the latter, it is instead possible to detect the concentration of sodium in the corresponding plasmatic water and from this value to trace back to the concentration in the blood. On this basis, it is frequent to use a device for measuring conductivity applied to a line for the plasmatic water, obtained for example via means for ultrafiltration of the blood, followed by means for purification by absorption, as described and claimed in the patent No. EP0451429, filed in the name of the present applicant.
As may appear obvious, having available a disposable device for measuring conductivity in contact with the plasmatic water leads to major problems of practicality, safety, and cost.

The aim of the present invention is to provide a circuit for extracorporeal dialysis, the technical characteristics of which are such as to overcome in a simple and economically advantageous way the problems of the known art referred to above.

The subject of the present invention is a circuit for extracorporeal dialysis, the essential characteristics of which are specified in Claim 1, and the preferred and/or auxiliary characteristics of which are specified in Claims 2-5.

The ensuing example serves an illustrative and non-limiting purpose for a better understanding of the invention with the aid of the figures of the annexed plate of drawings, wherein:
Figure 1 is a schematic view of a circuit for extracorporeal dialysis forming the subject of the present invention, with parts removed; and
Figure 2 is a lateral section of a detail of the circuit of Figure 1 coupled to a dialysis machine.

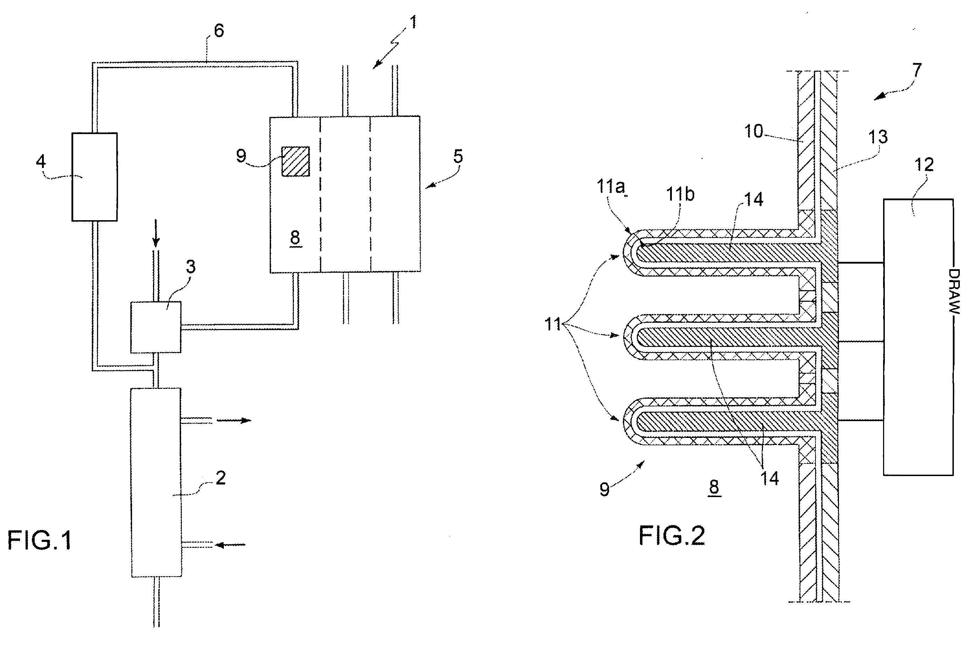

Designated as a whole by 1 in Figure 1 is a circuit for extracorporeal dialysis (illustrated only partially), of which there are schematically represented a dialysis filter 2, a unit for separation of the plasmatic water 3, a unit for purification by absorption 4, a cassette 5, and a line 6 for conduction of the plasmatic water, which, passing through the cassette 5 and the unit for purification by absorption 4, re-infuses the plasmatic water produced into the blood at inlet to the dialysis filter 2.

The circuit 1 is inserted within the machine 7, which is only partially illustrated in Figure 2.

Provided in the cassette 5 is a compartment 8 dedicated to the passage of the plasmatic water produced by the unit for separation of the plasmatic water 3 and, in effect, forming part of the conduction line 6.

The cassette 7 comprises a plurality of conductive elements 9 as illustrated schematically in Figure 1.

Illustrated in Figure 2 is a section of the conductive elements 9 mounted on an outer wall 10 of the cassette 5. Each of the conductive elements 9 is constituted by a hollow plug 11 made of conductive material and presenting a surface 11a facing the inside of the compartment 8 and hence in contact with the plasmatic water, and a surface 11b facing the outside of the cassette 5.

The dialysis machine 7 comprises a device 12 for measuring conductivity fixed to a panel 13 for covering the machine 7 itself. The device 12 for measuring conductivity comprises a plurality of electrodes 14, each of which engages a respective hollow plug 11 coming into contact with the surfaces 11b. As may seem obvious, the device 12 for measuring conductivity moreover comprises a set of electrical components (known and not illustrated) such as to be able to measure the conductivity and detect the value thereof.

In this way, it will be possible to make the measurements of conductivity without setting any material external to its own conduction line in contact with the plasmatic water, with the obvious advantages from the standpoint of safety that this entails. In fact, the measuring electrodes are the hollow plugs 11, which are fixed within the compartment 8.

According to a preferred embodiment, the dialysis machine forming the subject of the present invention comprises a device for measuring temperature (known and not illustrated for reasons of simplicity), which in turn comprises a sensor that can be inserted into one of the plugs 11, the characteristics of thermal and electrical conductivity of which guarantee the measurement of the temperature. Also in this case the measurement of the temperature of the plasmatic water is guaranteed without bringing into contact therewith parts external to its conduction line.

As may appear obvious to a person skilled of the art, the solution of the present invention, in addition to guaranteeing safety of monitoring, ensures a considerable practicality in so far as it is sufficient to mount the cassette 5 on the covering panel 13 to ensure the contacts necessary for obtaining a measurement of conductivity of the plasmatic water.

In the same way, the characteristics of the conductive element can be different from the ones described, albeit maintaining the presence of a first surface thereof facing inside the compartment of the plasmatic water, and a second surface thereof facing the outside of the cassette, for the purpose of guaranteeing the advantages of not bringing any external element into contact with the plasmatic water itself.

## Claims

1. A dialysis machine (7) comprising means for the separation of plasmatic water (3) from the patient's blood, and a line (6) for transport of the plasmatic water produced; the dialysis machine (7) being **characterized in that** it comprises a cassette (5), provided inside which is at least one compartment (8) isolated in a fluid-tight way and forming part of said line (6) for transport of the plasmatic water, and means for measuring the conductivity of the plasmatic water; said conductivity means comprising at least two conductive elements (9) fixed to a wall of said cassette (5) and presenting a first surface of contact (11a) facing the inside of said compartment (8) and designed to be in contact with the plasmatic water, and a second surface of contact (11b) facing the outside of the cassette (5), and a device for measuring conductivity (12), which can be coupled to said conductive elements (9) through said second surface of contact (11b).

2. The dialysis machine (7) according to Claim 1, **characterized in that** each of said conductive elements comprises a hollow plug (11) made of conductive material and presenting a surface (11a) facing the inside of said compartment (8) and a surface (11b) facing the outside of the cassette (5).

3. The dialysis machine (7) according to Claim 2, **characterized in that** said device for measuring conductivity comprises at least two electrodes (14), each of which is designed to be housed within a respective hollow plug (11).

4. The dialysis machine (7) according to Claim 3, **characterized in that** said electrodes are fixed to a panel (13) for covering said machine (7).

5. The dialysis machine (7) according to any one of the preceding claims, **characterized in that** it comprises a device for measuring temperature designed to use as sensor said conductive element (9)

## Patentansprüche

1. Dialysemaschine (7), umfassend Mittel zum Separieren von plasmatischem Wasser (3) von Blut eines Patienten, und eine Leitung (6) für den Transport des erzeugten plasmatischen Wassers; die Dialysemaschine (7) ist **dadurch gekennzeichnet, dass** sie eine Kassette (5) umfasst, wobei im Inneren wenigstens eine wasserdicht isolierte Kammer (8) vorgesehen ist, welche einen Teil der Leitung (6) für den Transport des plasmatischen Wassers bildet, und weiter Mittel zum Messen der Leitfähigkeit des plasmatischen Wassers umfasst; die Leilfahigkeits-Mittel umfassen wenigstens zwei leitende Elemente (9), die an einer Wand der Kassette (5) befestigt sind und eine erste Kontaktoberfläche (11a) darstellen, die dem Inneren der Kammer (8) zugewandt ist und die ausgelegt ist, um in Kontakt mit dem plasmatischen Wasser zu stehen, und weiter eine zweite Kontaktoberfläche (11b) darstellen, die dem Äußeren der Kassette (5) zugewandt ist, und eine Vorrichtung (12) für das Messen der Leitfähigkeit, die durch die zweite Kontaktoberfläche (11b) mit den leitenden Elementen (9) verbunden werden kann.

2. Dialysemaschine (7) nach Anspruch 1, **dadurch gekennzeichnet, dass** jedes der leitenden Elemente einen Hohlzapfen (11) umfasst, welcher aus einen leitenden Material besteht und eine Oberfläche (11a), die dem Inneren der Kammer (8) zugewandt ist, und eine Oberfläche (11 b), die dem Äußeren der Kassette (5) zugewandt ist, darstellt.

3. Dialysemaschine (7) nach Anspruch 2, **dadurch gekennzeichnet, dass** die Vorrichtung für das Messen der Leitfähigkeit wenigstens zwei Elektroden (14) umfasst, von denen jede ausgelegt ist, um in einem jeweiligen Hohlzapfen (11) aufgenommen, zu werden.

4. Dialysemaschine (7) nach Anspruch 3, **dadurch gekennzeichnet, dass** die Elektroden an einer Tafel (13) für das Verdecken der Maschine (7) befestigt sind.

5. Dialysemaschine (7) nach einem der vorstehenden Ansprüche, **dadurch gekennzeichnet, dass** sie eine Vorrichtung für das Messen einer Temperatur umfasst, die ausgelegt ist, um die leitenden Elemente (9) als Sensoren zu verwenden.

## Revendications

1. Machine de dialyse (7) comprenant un moyen pour la séparation d'eau plasmatique (3) du sang d'un patient, et une ligne (6) pour le transport de l'eau plasmatique produite ; la machine de dialyse (7) étant **caractérisée en ce qu'**elle comprend une cassette (5), prévue dedans qui est au moins un compartiment (8) isolé de manière étanche aux fluides et faisant partie de ladite ligne (6) pour le transport de l'eau plasmatique, et un moyen pour mesurer la conductivité de l'eau plasmatique ; ledit moyen de conductivité comprenant au moins deux éléments conducteurs (9) fixés à une paroi de ladite cassette (5) et présentant une première surface (11a) de contact en vis-à-vis de l'intérieur dudit compartiment (8) et conçue pour être en contact avec l'eau plasmatique, et une deuxième surface (11b) de contact en vis-à-vis de l'extérieur de la cassette (5), et un dispositif (12) de mesure de conductivité, qui peut être couplé auxdits éléments conducteurs (9) par l'intermédiaire de ladite deuxième surface (11b) de contact.

2. Machine de dialyse (7) selon la revendication 1, **caractérisée en ce que** chacun desdits éléments conducteurs comprend un bouchon (11) creux réalisé en un matériau conducteur et présentant une surface (11a) en vis-à-vis de l'intérieur dudit compartiment (8) et une surface (11b) en vis-à-vis de l'extérieur de la cassette (5).

3. Machine de dialyse (7) selon la revendication 2, **caractérisée en ce que** ledit dispositif de mesure de conductivité comprend au moins deux électrodes (14), dont chacune est conçue pour être logée dans un bouchon (11) creux respectif.

4. Machine de dialyse (7) selon la revendication 3, **caractérisée en ce que** lesdites électrodes sont fixées à une plaque (13) pour couvrir ladite machine (7).

5. Machine de dialyse (7) selon l'une quelconque des revendications précédentes, **caractérisée en ce qu'**elle comprend un dispositif de mesure de température conçu pour utiliser ledit élément conducteur (9) pour faire office de capteur.
